(19) Europäisches Patentamt
European Patent Office
Office européen des brevets

(11) **EP 3 994 453 B1**

(12) **EUROPÄISCHE PATENTSCHRIFT**

(45) Veröffentlichungstag und Bekanntmachung des Hinweises auf die Patenterteilung:
**20.03.2024  Patentblatt 2024/12**

(21) Anmeldenummer: **20739579.9**

(22) Anmeldetag: **01.07.2020**

(51) Internationale Patentklassifikation (IPC):
*G01N 21/88* (2006.01)     *G01N 21/95* (2006.01)
*G01N 21/55* (2014.01)     *G01J 3/46* (2006.01)
*G01N 21/27* (2006.01)

(52) Gemeinsame Patentklassifikation (CPC):
**G01N 21/8806; G01N 21/55; G01N 21/64;
G01N 21/95; G01N 33/36;** G01N 21/274;
G01N 2021/551; G01N 2021/8845;
G01N 2201/0221

(86) Internationale Anmeldenummer:
**PCT/EP2020/068551**

(87) Internationale Veröffentlichungsnummer:
**WO 2021/001437 (07.01.2021 Gazette 2021/01)**

(54) **PRÜFGERÄT UND VERFAHREN ZUR PRÜFUNG DER RETROREFLEXION UND/ODER FLUORESZENZ EINES OBJEKTS**

TEST DEVICE AND METHOD FOR TESTING THE RETROREFLECTION AND/OR FLUORESCENCE OF AN OBJECT

APPAREIL DE TEST ET PROCÉDÉ POUR TESTER LA RÉTRORÉFLEXION ET/OU LA FLUORESCENCE D'UN OBJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.07.2019   DE 102019117858**

(43) Veröffentlichungstag der Anmeldung:
**11.05.2022   Patentblatt 2022/19**

(73) Patentinhaber: **Diemietwaesche.De GmbH + Co. Kg
78052 Villingen-Schwenningen (DE)**

(72) Erfinder: **WEUTHEN, Michael
78559 Gosheim (DE)**

(74) Vertreter: **Rüger Abel Patentanwälte PartGmbB
Webergasse 3
73728 Esslingen a. N. (DE)**

(56) Entgegenhaltungen:
EP-A2- 2 184 600     DE-U1-202007 002 298
US-A- 5 828 460     US-A1- 2012 229 809

**Beschreibung**

Die Erfindung betrifft ein Handprüfgerät, das dazu eingerichtet ist, eine Retroreflexion und/oder eine Fluoreszenz eines Objekts zu prüfen, sowie ein Verfahren zum Durchführen einer solchen Prüfung. Bei dem Objekt handelt es sich insbesondere um Textilmaterial, wie Bekleidungsstücke und insbesondere um hochsichtbare Warnkleidung.

Bestimmte Objekte, insbesondere hochsichtbare Warnkleidung, müssen Anforderungen an eine Retroreflexion des Lichts, insbesondere weißen Lichts, und/oder an eine Fluoreszenz des Materials erfüllen. Für hochsichtbare Warnkleidung sind diese Anforderungen, z.B. in der Norm ISO 20471 definiert. Neben Warnkleidung gibt es auch andere Gegenstände, deren Sichtbarkeit von Bedeutung ist, wie etwa Warndreiecke, Baustellenmarkierungen, Verkehrsschilder, Markierungen an Fahrzeugen, insbesondere Einsatzfahrzeugen von Sicherheits- und Rettungskräften, usw.

EP 3 351 379 A1 beschreibt das Herstellen von fluoreszierenden Textilien. Nach der Herstellung werden die Farbeigenschaften entsprechend dem Xenon-Test gemäß Abschnitt 5.2 der ISO 20471 geprüft und die Leuchtdichte nach einer Xenon-Bestrahlung gemessen.

Ein portables Farbmessgerät wird durch die Firma HunterLab unter der eingetragenen Marke *"MiniScan® EZ"* angeboten. Für die Durchführung des Xenon-Tests weist dieses Gerät eine Xenon-Blitzlampe auf. Die Farbmessung soll dadurch CIE-konform sein und hochgenaue Spektraldaten liefern.

Aus einer Vortragsveranstaltung der Firma 3M am 17. November 2014 unter dem Titel *"Innovation trifft Industrie"* ist ein Verfahren zur Leuchtdichtevermessung bekannt, bei dem eine Lichtquelle und ein Leuchtdichtemessgerät in einem Abstand von ca. 20 m zu einer Puppe angeordnet sind. An der Puppe kann Warnkleidung angebracht werden. Die Puppe kann um 360° um die Hochachse gedreht werden, so dass die Warnkleidung über diese Anordnung ringsum vermessen werden kann.

EP 3 327 627 beschreibt ein Verfahren und eine Vorrichtung zur visuellen Qualitätskontrolle von Textilien. Dazu wird wenigstens ein Bild des Textilobjekts aufgenommen und ein Teilbereich des Bildes ausgewählt. Die Bildpunkte in diesem Teilbereich werden mit einem vorgegebenen Bewertungskriterium ausgewertet und daraus eine Bewertungsgröße ermittelt. Die Bewertungsgröße wird mit einem Schwellenwert verglichen, wobei der Vergleich das Ergebnis der Qualitätskontrolle darstellt.

US 5 828 460 A offenbart eine Vorrichtung zur optischen Prüfung von metallischen Oberflächen. Die Vorrichtung hat mehrere Lichtquellen zur Beleuchtung der metallischen Oberfläche. Über Fotosensoren wird reflektiertes Licht empfangen. Die Vorrichtung strahlt zunächst UV-Licht auf die metallische Oberfläche, um festzustellen, ob Öl auf der Oberfläche anhaftet. Befinden sich keine größeren Mengen Öl auf der metallischen Oberfläche, wird sichtbares Licht abgestrahlt, dessen Reflexion dann von einem anderen Fotosensor empfangen wird.

Eine ähnliche Vorrichtung ist auch in US 2012/0229809 A1 beschrieben. Ein Spektrometer mit einer Hauptlichtquelle strahlt Licht mit einem breiten Lichtspektrum auf eine Objektoberfläche ab, wobei das Spektrum des reflektierten Lichts untersucht wird. Das Gerät ist auch in der Lage, eine Fluoreszenzprüfung durchzuführen.

Die eingangs genannten Objekte sind einem gewissen Verschleiß unterworfen. Zum Beispiel kann das Material der Warnbekleidung durch das Waschen einem Verschleißt unterliegen. Dies kann die Sichtbarkeit beeinträchtigen. Es besteht daher ein Bedarf zu prüfen, ob die genannten Gegenstände die vorgegebenen Anforderungen im Hinblick auf die Sichtbarkeit erfüllen. Die bisherigen Vorrichtungen und Verfahren sind aufwendig und/oder benötigen teure Vorrichtungen. Es kann als Aufgabe der vorliegenden Erfindung angesehen werden, eine Prüfung der Retroreflexion und/oder Fluoreszenz eines Objekts zu ermöglichen, die sehr schnell und einfach auch durch wenig geschultes oder ungeschultes Personal durchführbar ist.

Diese Aufgabe wird durch ein Handprüfgerät mit den Merkmalen des Patentanspruches 1 sowie ein Verfahren mit den Merkmalen des Patentanspruches 15 gelöst.

Das erfindungsgemäße Handprüfgerät ist dazu eingerichtet, in wenigstens zwei verschiedenen Prüfmodi betrieben zu werden. In einem Reflexionsprüfmodus kann eine Retroreflexion eines Objekts geprüft werden. In wenigstens einem Fluoreszenzprüfmodus kann eine Fluoreszenz eines Objekts geprüft werden. Insbesondere stehen für unterschiedlich farbige fluoreszierende Objekte mehrere Fluoreszenzprüfmodi zur Verfügung, beispielsweise ein Fluoreszenzprüfmodus für ein gelb fluoreszierendes Objekt und ein Fluoreszenzprüfmodus für ein orange fluoreszierendes Objekt. Zusätzlich oder alternativ können auch andere oder weitere Fluoreszenzprüfmodi für andere oder weitere Farben vorhanden sein, beispielsweise für ein rot fluoreszierendes Objekt und/oder ein grün fluoreszierendes Objekt.

Das Handprüfgerät ist insbesondere dazu eingerichtet, Textilmaterial aufweisende Objekte, wie Kleidungsstücke, zu prüfen. Das Handprüfgerät kann insbesondere dazu eingerichtet und/oder kalibriert sein, hochsichtbare Warnkleidung gemäß ISO 20471 zu prüfen.

Das Handprüfgerät hat ein Gehäuse mit einer Bedienschnittstelle zur Auswahl des Prüfmodus. Die Bedienschnittstelle kann bei einem Ausführungsbeispiel außerdem ein optisches und/oder akustisches Ausgabemittel zur Ausgabe des Prüfergebnisses an eine Bedienperson aufweisen. Als optisches Ausgabemittel können wenigstens ein Signalleuchtmittel und/oder ein Display oder eine andere beliebige Anzeige verwendet werden.

An einer Vorderseite hat das Gehäuse ein Fenster. Das Fenster ist zumindest in einem vorgegebenen Wellenlängenbereich lichtdurchlässig. Beim Ausführungsbeispiel ist das Fenster zumindest für die Lichtwellenlängen lichtdurchlässig, die von wenigstens einer Leuchtdiode einer Prüfanordnung des Handprüfgeräts emittiert werden und für Lichtwellenlängen im sichtbaren Bereich. Abgesehen von dem Fenster ist das Gehäuse ansonsten im Wesentlichen lichtdicht verschlossen, so dass Licht im Wesentlichen ausschließlich durch das Fenster in den Innenraum des Gehäuses einfallen kann.

In dem Gehäuse befindet sich die Prüfanordnung mit einer und vorzugsweise genau einer Weißlicht-Leuchtdiode und einer und insbesondere genau einer UV-Leuchtdiode. Die Prüfanordnung weist außerdem eine Empfangsschaltung mit einem und insbesondere genau einem Fotoempfänger sowie eine Steuereinheit auf. Die Steuereinheit ist zur Ansteuerung der Weißlicht-Leuchtdiode und der UV-Leuchtdiode eingerichtet. Die Steuereinheit ist mit der Empfangsschaltung kommunikationsverbunden und dazu eingerichtet, ein Empfangssignal der Empfangsschaltung zu empfangen. Der Fotoempfänger ist für einfallendes Licht mit einer Wellenlänge von kleiner als 400 nm nicht sensitiv.

Die Weißlicht-Leuchtdiode und der Fotoempfänger sind derart im Gehäuse angeordnet, dass von der Weißlicht-Leuchtdiode emittiertes weißes Licht durch das Fenster auf das zu prüfende Objekt auftrifft, dort reflektiert wird, wieder durch das Fenster eintritt und am Fotoempfänger empfangen wird. Weißes Licht legt diesen Lichtweg zurück, wenn ein zu prüfendes Objekt vor oder an der Vorderseite des Gehäuses vorhanden ist und der Reflexionsprüfmodus ausgewählt und gestartet wurde.

Die UV-Leuchtdiode und der Fotoempfänger sind derart angeordnet, dass von der UV-Leuchtdiode emittiertes ultraviolettes Licht durch das Fenster auf das Objekt auftrifft und dort fluoreszierendes Material anregt. Das aufgrund der Anregung vom Objekt emittierte Licht tritt durch das Fenster ein und wird am Fotoempfänger empfangen. Ultraviolettes Licht legt diesen Lichtweg zurück, wenn sich vor der Vorderseite oder an der Vorderseite des Gehäuses ein Objekt mit fluoreszierendem Material befindet und der wenigstens eine Fluoreszenzprüfmodus ausgewählt und gestartet wurde.

Bevorzugt hat der Fotoempfänger eine optische Achse, die das Fenster durchsetzt, insbesondere mittig durchsetzt. Die optische Achse des Fotoempfängers kann im Wesentlichen orthogonal zur Ebene des Fensters ausgerichtet sein.

Die Weißlicht-Leuchtdiode kann parallel zur optischen Achse des Fotoempfängers versetzt angeordnet sein, um Direkteinstrahlung von weißem Licht auf den Fotoempfänger zu vermeiden. Insbesondere befindet sich die Weißlicht-Leuchtdiode näher am Fenster als der Fotoempfänger.

Die Steuereinheit ist erfindungsgemäß dazu eingerichtet, im Reflexionsprüfmodus die Weißlicht-Leuchtdiode und im wenigstens einen Fluoreszenzprüfmodus die UV-Leuchtdiode zur Lichtemission anzusteuern. Ein Empfangssignal der Empfangsschaltung beschreibt die Bestrahlungsstärke des auf den Fotoempfänger auftreffenden Lichts. Das Empfangssignal wird durch die Steuereinheit ausgewertet, wobei die Auswertung dem Prüfergebnis entspricht. Das Prüfergebnis kann der Bedienperson über der Bedienschnittstelle ausgegeben bzw. angezeigt werden. Im einfachsten Fall kann der Bedienperson ausgegeben oder angezeigt werden, ob das geprüfte Objekt die Anforderungen an die Retroreflexion eines retroreflektierenden Materials oder die Anforderungen an die Leuchtdichte eines fluoreszierenden Materials erfüllt. Hierzu kann das Empfangssignal beispielsweise mit einem Referenzwert verglichen werden. Der Referenzwert kann, z.B. ein Mindestwert sein, der erfüllt werden muss.

Alternativ oder zusätzlich dazu kann auch eine Ausgabe erfolgen, die einen Anteil oder ein Verhältnis am vorgegebenen Referenzwert angibt. Diese Anzeige kann zum Beispiel eine Prozentangabe sein.

Somit ist erfindungsgemäß ein genaues Ermitteln eines Messwertes, z.B. für eine Leuchtdichte, nicht notwendig. Mit der Erfindung können sowohl die Retroreflexion als auch die Fluoreszenz eines Objekts, insbesondere von Warnkleidung, auf sehr einfache Weise geprüft werden. Die Prüfdauer ist extrem kurz und liegt im Bereich von wenigen Sekunden. Wird beispielsweise Warnkleidung in einer Wäscherei gewaschen und nach dem Waschen geprüft, kann eine Prüfung sämtlicher gewaschenen Kleidungsstücke in kurzer Zeit erfolgen. Dadurch kann sichergestellt werden, dass gebrauchte und mehrfach gewaschene Warnkleidung noch eine ausreichende Sicherheit für die Person bietet, die die Warnkleidung trägt.

Das Handprüfgerät weist einen sehr einfachen Aufbau auf und kann kostengünstig realisiert werden. Eine Weißlicht-Leuchtdiode, eine UV-Leuchtdiode und ein Fotoempfänger sind als optische Bestandteile der Prüfanordnung des Handprüfgeräts bereits ausreichend. Die elektrische und/oder elektronische Schaltung der Prüfanordnung kann mit einfachen und kostengünstigen Standardbauteilen aufgebaut werden.

Die Steuereinheit ist mit der Bedienschnittstelle kommunikationsverbunden. Bei einer bevorzugten Ausführungsform weist die Bedienschnittstelle wenigstens eine Eingabetaste und/oder eine Anzeige auf. Beispielsweise kann die Bedienschnittstelle für jeden auswählbaren Prüfmodus eine Eingabetaste aufweisen.

Bevorzugt ist die Steuereinheit dazu eingerichtet, das Empfangssignal mit einem Referenzwert zu vergleichen. Der Referenzwert kann im Rahmen einer Kalibrierung des Handprüfgeräts vorgegeben werden. Das Vergleichsergebnis gibt insbesondere an, ob das Objekt im Hinblick auf die Retroreflexion und/oder die Fluoreszenz einer Vorgabe entspricht oder gibt an, wie das Verhältnis zwischen dem aktuell geprüften Objekt und dem vorgegebenen Referenzwert im betreffenden Prüfmodus ist.

Zur Ermittlung und/oder Vorgabe des Referenzwertes kann das Handprüfgerät selbst verwendet werden. Beispielsweise kann mittels eines Kalibrierobjekts oder einer Kalibriereinrichtung, dessen bzw. deren Eigenschaften bekannt sind, eine Kalibrierung erfolgen. Bei der Prüfung eines bekannten Kalibrierobjekts kann das Empfangssignal den Referenzwert bilden, der beispielsweise ein Mindestwert für die Retroreflexion (Reflexionsprüfmodus) oder ein Mindestwert für die Fluoreszenz (Fluoreszenzprüfmodus) charakterisiert. Die anschließenden Prüfungen von zu prüfenden Objekten können dann ermitteln, ob die Fluoreszenz bzw. die Retroreflexion eines geprüften Objekts zumindest den jeweils zugeordneten Referenzwert erreicht und/oder wie groß das Verhältnis der Fluoreszenz bzw. der Retroreflexion eines geprüften Objekts zu dem jeweils zugeordneten Referenzwert ist. Zum Beispiel kann das Verhältnis als Prozentangabe über die Bedienschnittstelle ausgegeben werden.

Alternativ zu einem Kalibrierobjekt kann beispielsweise auch eine lichtabstrahlende Kalibriereinrichtung eingesetzt werden, bei der die Leuchtdichte und/oder die Wellenlänge des abgestrahlten Lichts bekannt oder einstellbar ist. Mit Hilfe einer solchen Kalibriereinrichtung kann die Empfangsschaltung ohne Verwendung der Leuchtdioden des Handprüfgerätes kalibriert werden.

Es ist auch möglich, für jeden Prüfmodus einen entsprechenden Kalibriermodus vorzusehen, der von einem autorisierten Bediener aktiviert werden kann. Dadurch kann das Handprüfgerät nicht nur einmalig im Rahmen der Herstellung, sondern auch später erneut zu kalibrieren, beispielsweise um Veränderungen in der Charakteristik der Leuchtdioden und/oder des Fotoempfängers kompensieren zu können oder um das Handprüfgerät an aktuelle Umgebungsbedingungen anzupassen.

Es ist auch möglich, zusätzlich zu dem Referenzwert einen oder mehrere weitere Kalibrierwerte zu erzeugen bzw. vorzugeben und in der Steuereinheit abzuspeichern. Dadurch kann beispielsweise ein Kennfeld, eine Kennlinie oder eine andere Charakteristik erzeugt werden. Durch das Hinterlegen von zusätzlichen Kalibrierwerten lässt sich zum Beispiel auch ein nichtlineares Verhalten berücksichtigen. Die Genauigkeit kann erhöht werden.

Wie bereits erläutert, kann das Vergleichsergebnis als Prüfergebnis über die Bedienschnittstelle und insbesondere über die Anzeige ausgegeben werden. Bei einer bevorzugten Ausführungsform wird ein Verhältnis des Empfangssignals zum Referenzwert des aktuell verwendeten Prüfmodus angegeben.

Es ist vorteilhaft, wenn das Gehäuse an der Vorderseite eine Anlagefläche aufweist, die dazu eingerichtet ist, während der Prüfung am Objekt angelegt zu werden. Die Anlagefläche kann das Fenster vollständig umschließen. Dadurch wird Streulichteinfall durch das Fenster vermieden und ein definierter Abstand des Fotoempfängers und der Leuchtdiode zum Objekt vorgegeben. Das Fenster bzw. eine Abdeckplatte des Fensters kann dabei Bestandteil der Anlagefläche sein oder benachbart zur Anlagefläche und insbesondere gegenüber der Anlagefläche zurückgesetzt angeordnet sein.

Vorzugsweise kann die Anlagefläche lichtundurchlässige, elastische Bestandteile aufweisen, die das Fenster vollständig umschließen. Dadurch können Unebenheiten im zu prüfenden Objekt ausgeglichen werden.

Die Empfangsschaltung weist bei einem bevorzugten Ausführungsbeispiel einen Transmissionsverstärker auf. Der Fotoempfänger kann an einen Eingang des Transmissionsverstärkers angeschlossen sein. An einem Ausgang des Transmissionsverstärkers liegt das Empfangssignal an. Der Transmissionsverstärker kann einen einstellbaren Widerstand aufweisen, um den Wertebereich und insbesondere einen Spannungsbereich für das Empfangssignal einzustellen. Beispielsweise kann das Empfangssignal im Bereich von 0-5 Volt variieren, abhängig von der Bestrahlungsstärke des auf den Fotoempfänger auftreffenden Lichts.

Der Fotoempfänger hat eine relative spektrale Sensitivität, derart, dass er keinen oder lediglich einen unerheblich kleinen Fotostrom erzeugt, wenn ausschließlich ultraviolettes Licht auf den Fotoempfänger auftrifft. Somit kann ultraviolettes Streulicht, das von der UV-Leuchtdiode emittiert wird, die vom Fotoempfänger im wenigstens einen Fluoreszenzprüfmodus ermittelte Bestrahlungsstärke nicht oder nur unwesentlich beeinträchtigen. Der Fotoempfänger ist nicht sensitiv für einfallendes Licht mit einer Wellenlänge von kleiner als 400 nm.

Die Steuereinheit kann dazu eingerichtet sein, die Weißlicht-Leuchtdiode und/oder die UV-Leuchtdiode für die Prüfung für eine vorgegebene Emissionszeitdauer zu aktivieren. Die Emissionszeitdauer ist insbesondere kleiner als 1,0 Sekunden. Durch die kurze Emissionszeitdauer entstehen keine thermischen Beeinflussungen durch Wärmestrahlung auf das zu prüfende Objekt.

Die Prüfung eines Objekts wird wie folgt durchgeführt:

Das Handprüfgerät wird mit dem Fenster in Richtung des Objekts angeordnet. Anschließend wird ein Prüfmodus über die Bedienschnittstelle ausgewählt. Vorzugsweise startet die Steuereinheit den ausgewählten Prüfmodus automatisch. Die Steuereinheit aktiviert im Reflexionsprüfmodus die Weißlicht-Leuchtdiode und im wenigstens einen Fluoreszenzprüfmodus die UV-Leuchtdiode. Das dadurch in der Empfangsschaltung erzeugte Empfangssignal wird in der Steuereinheit ausgewertet. Insbesondere wird es mit einem vorgegebenen Referenzwert verglichen. Das Vergleichsergebnis kann über die Bedienschnittstelle ausgegeben werden, beispielsweise akustisch und/oder optisch.

Vorteilhafte Ausgestaltungen der Erfindung ergeben sich aus den abhängigen Ansprüchen, der Beschreibung sowie den Zeichnungen. Nachfolgend werden bevorzugte Ausführungsbeispiele der Erfindung anhand der beigefügten Zeichnungen im Einzelnen erläutert. Es zeigen:

Figur 1 eine schematische, blockschaltbildähnliche Darstellung eines Ausführungsbeispiels eines Handprüfgeräts,

Figur 2 eine schematische Darstellung eines zu prüfenden Objekts, das beispielsgemäß durch ein Warnkleidungsstück gebildet ist,

Figur 3 eine schematische Darstellung der Verwendung des Handprüfgeräts aus Figur 1 bei der Prüfung des Objekts aus Figur 2 in einem Reflexionsprüfmodus,

Figur 4 eine schematische Darstellung der Verwendung des Handprüfgeräts aus Figur 1 bei der Prüfung des Objekts aus Figur 2 in einem Fluoreszenzprüfmodus,

Figur 5 einen Schaltplan eines Ausführungsbeispiels einer Prüfanordnung des Handprüfgeräts,

Figur 6 eine Kennlinie, mittels der eine Auswertung eines bei der Prüfung erzeugten Empfangssignals erfolgen kann und

Figur 7 eine relative spektrale Sensitivität eines Ausführungsbeispiels eines Fotoempfängers des Handprüfgeräts.

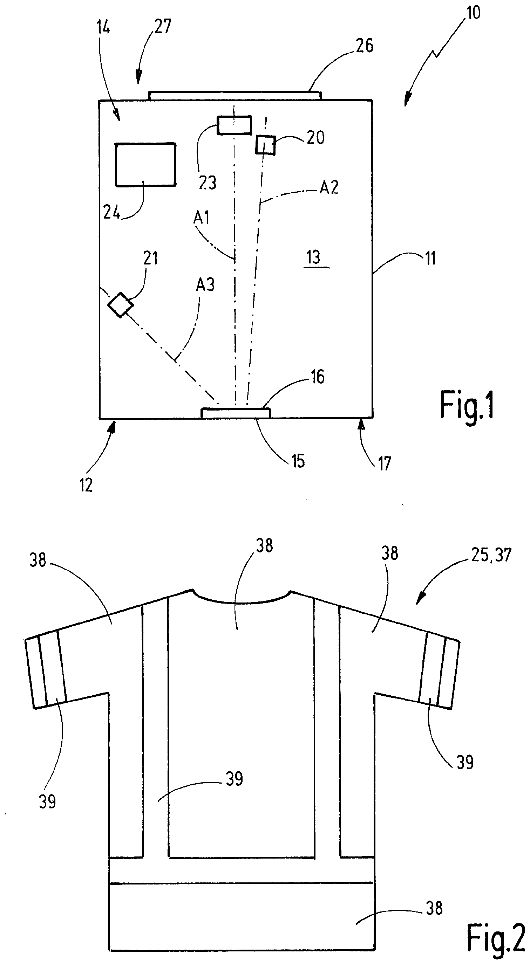

In Figur 1 ist schematisch nach Art eines Blockschaltbilds ein Ausführungsbeispiel eines Handprüfgeräts 10 veranschaulicht. Das Handprüfgerät 10 hat ein Gehäuse 11 mit einer Vorderseite 12. Das Gehäuse 11 begrenzt einen Innenraum 13, in dem eine Prüfanordnung 14 des Handprüfgeräts 10 angeordnet ist. An der Vorderseite 12 hat das Gehäuse 11 ein Fenster 15, durch das Licht zumindest in einem vorgegebenen Lichtwellenbereich in den Innenraum 13 einfallen kann. Das Fenster 15 ist beispielsgemäß durch eine von einer lichtdurchlässigen Platte 16 abgedeckten Durchbrechung im Gehäuse 11 gebildet. Beim Ausführungsbeispiel ist die Platte 16 zumindest für Licht im ultravioletten Wellenlängenbereich sowie im sichtbaren Wellenlängenbereich durchlässig. Die Platte 16 bzw. das Fenster 15 können somit zumindest in einem Lichtwellenlängenbereich von 300 oder 350 nm bis 700 nm durchlässig sein. Das Fenster 15 bzw. die Platte 16 können auch für sämtliche Lichtwellenlängen transparent sein.

Licht gelangt in den Innenraum 13 beispielsgemäß ausschließlich über das Fenster 15. Im Übrigen ist der Innenraum 13 durch das Gehäuse 11 gegen Lichteinfall verschlossen.

An der Vorderseite 12 hat das Gehäuse 11 eine Anlagefläche 17. Die Anlagefläche 17 umschließt das Fenster 15 vollständig. Das Fenster 15 kann gegenüber der Anlagefläche in Richtung zum Fotoempfänger 23 hin zurückgesetzt sein und/oder in der Ebene der Anlagefläche 17 angeordnet sein, so dass die nach außen gewandte Fläche der Platte 16 ein Bestandteil der Anlagefläche 17 ist.

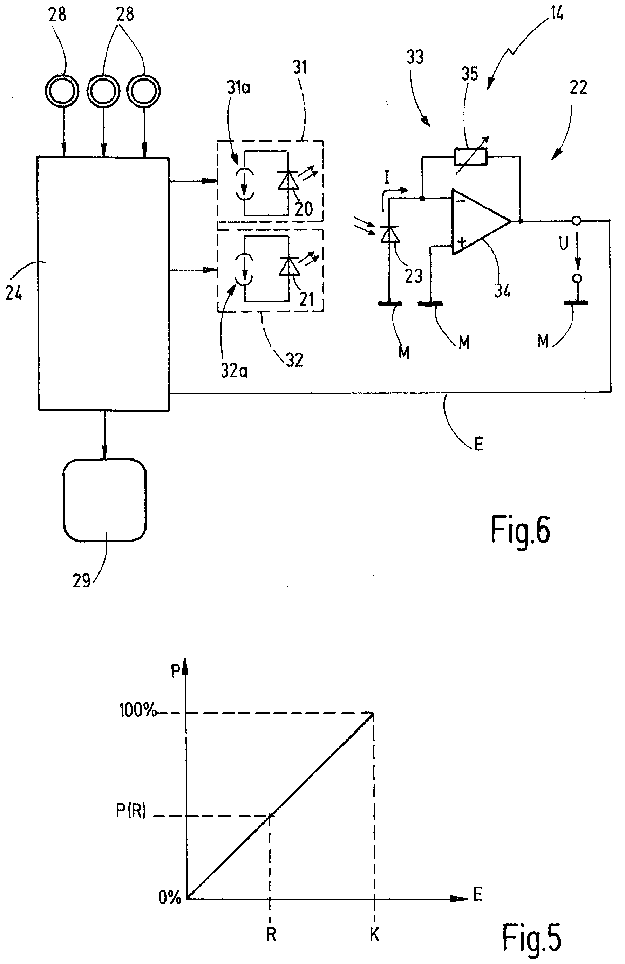

Die Prüfanordnung 14 des Handprüfgeräts 10 weist eine Weißlicht-Leuchtdiode 20, eine UV-Leuchtdiode 21, eine Empfangsschaltung 22 (Figur 5) mit einem Fotoempfänger 23 sowie eine Steuereinheit 24 auf. Die Weißlicht-Leuchtdiode 20 ist dazu eingerichtet, weißes Licht zu emittieren, beispielsweise in einem Wellenlängenbereich von etwa 400 nm bis etwa 700 nm. Durch die Weißlicht-Leuchtdiode 20 kann Licht emittiert werden, das vorzugsweise sämtlichen Wellenlängen enthält, die auch vom menschlichen Auge erfassbar sind.

Der Fotoempfänger 23 hat eine optische Achse A1, die das Fenster 15 durchsetzt, beispielsweise in etwa mittig. Die optische Achse A1 des Fotoempfängers 23 kann rechtwinklig zur Öffnung bzw. zur Fensterebene des Fensters 15 und beispielsgemäß rechtwinklig zur Platte 16 ausgerichtet sein.

Die Weißlicht-Leuchtdiode 20 hat eine optische Achse A2, die geneigt zur optischen Achse A1 des Fotoempfängers 23 angeordnet ist, beispielsweise in einem Winkel von kleiner als 10° und vorzugsweise in einem Winkel von kleiner als 8°. Beim Ausführungsbeispiel schließen die optische Achse A2 der Weißlicht-Leuchtdiode 20 und die optische Achse A1 des Fotoempfängers 23 einen Winkel von 7,5° ein. Die optische Achse A2 der Weißlicht-Leuchtdiode 20 und die optische Achse A1 des Fotoempfängers 23 schneiden sich vorzugsweise in einem Punkt, der in der Ebene der Anlagefläche 17 an der Vorderseite 12 des Gehäuses 11 liegt oder nahe zu dieser der Ebene angeordnet ist. An dieser Stelle ist bei einer Prüfung eine Oberfläche eines zu prüfenden Objekts 25 angeordnet.

Eine optische Achse A3 der UV-Leuchtdiode 21 schließt mit der optischen Achse A1 des Fotoempfängers 23 vorzugsweise einen Winkel von 45° ein.

Sämtliche optische Achsen A1, A2, A3 durchsetzen das Fenster 15 bzw. die Platte 16, wie es schematisch in Figur 1 veranschaulicht ist.

Das Handprüfgerät 10 ist dazu eingerichtet, unterschiedliche Prüfmodi auszuführen. Über die Steuereinheit 24 wird der Betrieb im ausgewählten Prüfmodus ausgeführt. Die Steuereinheit 24 ist mit einer Bedienschnittstelle 26 kommunikationsverbunden. Die Bedienschnittstelle 26 ist von außen zugänglich am Gehäuse 11 angeordnet, beispielsweise auf einer der Vorderseite 12 entgegengesetzten Rückseite 27 des Gehäuses. Beim Ausführungsbeispiel weist die Bedienschnittstelle 26 für jeden möglichen Prüfmodus eine Auswahltaste 28 auf und hat ein Mittel zur Ausgabe eines Prüfergebnisses, beispielsweise eine Anzeige 29, die als Bildschirm (z.B. LED-, OLED- oder LCD- Bildschirm) ausgebildet sein kann. Alternativ oder zusätzlich können auch andere oder weitere akustische und/oder optische Ausgabemittel vorhanden sein.

In Figur 5 ist ein Schaltungsaufbau für die Prüfanordnung 24 in Form eines Prinzipschaltplans veranschaulicht. Die Steuereinheit 24 ist mit der Bedienschnittstelle 26 und beispielsgemäß den Auswahltasten 28 sowie der Anzeige 29 kommunikationsverbunden. Außerdem ist die Steuereinheit mit einem Betriebsschaltkreis 31 für die Weißlicht-Leuchtdiode 20 und einem Betriebsschaltkreis 32 für die UV-Leuchtdiode 21 kommunikationsverbunden. Jeder Betriebsschaltkreis 31, 32 weist eine ansteuerbare Stromquelle 31a bzw. 32a und/oder einen ansteuerbaren Schalter auf, so dass durch die Ansteuerung mittels der Steuereinheit 24 ein Stromfluss durch die Weißlicht-Leuchtdiode 20 bzw. die UV-Leuchtdiode 21 bewirkt und/oder dessen Betrag gesteuert werden kann.

Bei der ersten Inbetriebnahme des Handprüfgerätes 10 hat der Strom durch die betreffende Leuchtdiode 20, 21 in dem jeweiligen Betriebsschaltkreis 31, 32 einen Betrag von weniger als 100% oder maximal 90% oder maximal 80% des maximalzulässigen Betriebsstromes der betreffenden Leuchtdiode 20, 21. Durch Erhöhung des Stromes - z.B. mittels der jeweiligen ansteuerbaren Stromquelle 31a, 32a - können Degradationseffekte der Leuchtdioden 20, 21, die mit zunehmender Gesamtbetriebsdauer auftreten können, zumindest teilweise ausgeglichen werden.

Die Empfangsschaltung 22 weist beim Ausführungsbeispiel einen Transmissionsverstärker 33 auf, an dessen Eingang der Fotoempfänger 23 angeschlossen ist und an dessen Ausgang ein Empfangssignal E bereitgestellt wird, das beispielsgemäß in Form einer Ausgangsspannung U bezüglich eines Massepotentials M anliegt. Der Transmissionsverstärker hat beispielsgemäß einen Operationsverstärker 34, an dessen invertierendem Eingang der Fotoempfänger 23 angeschlossen ist. Beim Ausführungsbeispiel ist der Fotoempfänger 23 durch eine Fotodiode gebildet, deren Kathode mit dem invertierenden Eingang des Operationsverstärkers 34 und deren Anode mit dem Massepotential M verbunden ist. Abhängig von der Bestrahlungsstärke liefert der Fotoempfänger 23 einen Fotostrom I. Zwischen den invertierenden Eingang des Operationsverstärkers 34 und den Ausgang des Operationsverstärkers 34, an dem die Ausgangsspannung U anliegt, ist ein Widerstand und beispielsgemäß ein einstellbarer Widerstand 35 geschaltet. Der einstellbare Widerstand 35 kann z.B. durch ein Potenziometer gebildet werden. Über den einstellbaren Widerstand 35 lässt sich der Spannungsbereich der Ausgangsspannung U anpassen. Für die Ausgangsspannung U gilt:

$$U = -R \cdot I,$$

wobei R der Betrag des ohmschen Widerstands des einstellbaren Widerstands 35 ist und I der vom Fotoempfänger 23 erzeugte Fotostrom ist.

Das Empfangssignal E, beispielsgemäß gebildet durch die Ausgangsspannung U, wird an die Steuereinheit 24 übermittelt. Die Empfangsschaltung 22 kann alternativ zum dargestellten Ausführungsbeispiel weitere Schaltungsteile zur Aufbereitung des Fotostromes I aufweisen, um ein Empfangssignal E zu erzeugen, das von der Steuereinheit 24 empfangen und ausgewertet werden kann. Bei dem hier veranschaulichten Ausführungsbeispiel wird der einstellbare Widerstand 35 derart justiert, dass die Ausgangsspannung U abhängig vom Betrag des Fotostromes I in einem Bereich von 0 V bis 5 V variiert.

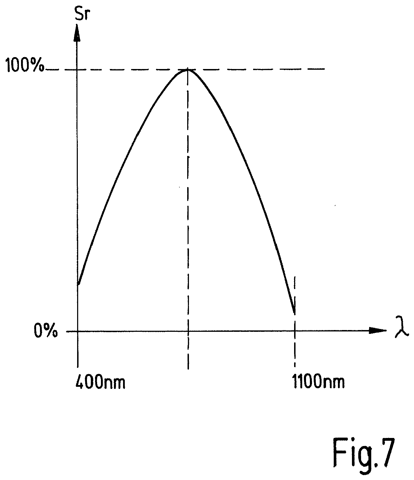

In Figur 7 ist eine beispielhafte relative spektrale Sensitivität Sr für einen Fotoempfänger 23 veranschaulicht. Vorzugsweise ist die relative spektrale Sensitivität Sr derart gewählt, dass der Fotoempfänger 23 keinen oder lediglich einen unerheblich geringen Fotostrom I erzeugt, wenn ultraviolettes Licht auf den Fotoempfänger 23 auftrifft. Beim hier veranschaulichten Ausführungsbeispiel liefert der Fotoempfänger 23 einen Fotostrom I größer als 0, wenn Licht im Wellenlängenbereich von mindestens 400 nm bis maximal etwa 1100 nm auf die sensitive Fläche des Fotoempfängers 23 auftrifft. Dadurch, dass ultraviolettes Licht keinen oder lediglich einen unerheblichen Fotostrom I bewirkt, wird die Prüfung durch Streulicht der UV-Leuchtdiode 21 nicht beeinflusst. Um den Einfluss von weißem Streulicht der Weißlicht-Leuchtdiode 20 zu vermeiden, ist diese in Richtung der optischen Achse A1 des Fotoempfängers 23 zum Fenster 15 hin versetzt, so dass eine Streulichtabstrahlung auf den Fotoempfänger 23 vermieden oder derart reduziert ist, dass sie den Fotostrom I nicht wesentlich beeinflusst.

Bei dem hier veranschaulichten Beispiel ist das zu prüfende Objekt 25 ein Warnbekleidungsstück 37. Mit dem Handprüfgerät 10 kann geprüft werden, ob das Warnbekleidungsstück 37 vorgegebene Erfordernisse erfüllt, insbesondere solche Erfordernisse, die in der Norm ISO 20471 vorgegeben sind. Das Warnbekleidungsstück 37 hat beim Ausführungsbeispiel wenigstens einen fluoreszierenden Bereich 38 und wenigstens einen retroreflektierenden Bereich 39. Vorzugsweise ist das gesamte Warnbekleidungsstück 37 aus einem fluoreszierenden textilen Trägermaterial hergestellt und retroflektierende Aufsätze, beispielsweise Streifen, sind auf das fluoreszierende textilen Trägermaterial aufgebracht.

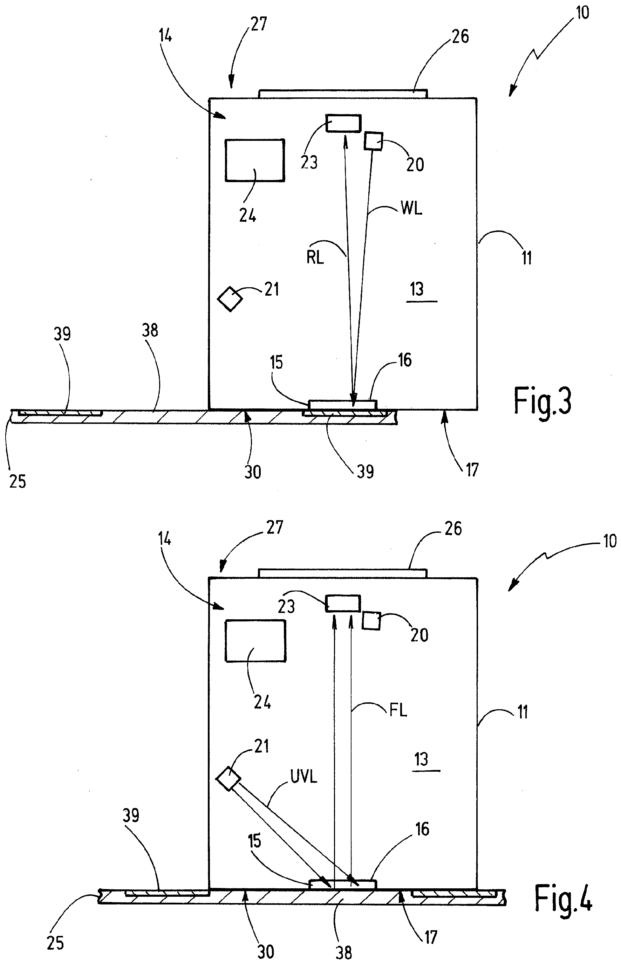

Mithilfe des Handprüfgeräts 10 kann sowohl die Retroreflexion des wenigstens einen retroreflektierenden Bereichs 39 als auch die Fluoreszenz des wenigstens einen fluoreszierenden Bereichs 38 geprüft werden. Hierzu weist das Handprüfgerät 10 unterschiedliche Prüfmodi auf. In einem Reflexionsprüfmodus wird die Retroreflexion eines retroreflektierenden Bereichs 39 (Figur 3) und in wenigstens einem Fluoreszenzprüfmodus die Fluoreszenz eines fluoreszierenden Bereichs 38 (Figur 4) geprüft. Beim Ausführungsbeispiel ist das Handprüfgerät dazu eingerichtet, die Fluoreszenz sowohl von einem gelb fluoreszierenden Material, als auch von einem orange fluoreszierenden Material prüfen zu können und weist hierfür jeweils einen separaten Fluoreszenzprüfmodus auf. Somit sind beim Ausführungsbeispiel drei unterschiedliche Prüfmodi vorhanden. Abweichend hierzu können auch mehr oder weniger Prüfmodi vorhanden sein, beispielsweise noch ein zusätzlicher Fluoreszenzprüfmodus für rot fluoreszierendes Material.

In Figur 3 ist schematisch die Durchführung einer Prüfung im Retroreflexionsmodus veranschaulicht. Zur Prüfung eines retroreflektierenden Bereichs 39 wird das Handprüfgerät 10 mit der Anlagefläche 17 derart auf das Objekt 25 aufgelegt, dass das Fenster an dem zu prüfenden retroreflektierenden Bereich 39 anliegt oder diesen unmittelbar gegenüber liegt. Dadurch, dass die Anlagefläche 17 am Objekt 25 anliegt, wird der Eintritt von Streulicht aus der Umgebung in das Fenster 15 vermieden.

Die Steuereinheit 24 aktiviert die Weißlicht-Leuchtdiode 20 nach dem Auswählen des Reflexionsprüfmodus über die Bedienschnittstelle 26 für eine kurze Emissionszeitdauer von beispielsweise weniger als 1 Sekunde. Die Weißlicht-Leuchtdiode 20 emittiert während der Emissionszeitdauer weißes Licht WL, das durch das Fenster 15 austritt und auf den retroreflektierenden Bereich 39 des Objekts 25 auftrifft. Dort wird das weiße Licht WL reflektiert und trifft als reflektiertes Licht RL wieder in das Fenster 15 ein und trifft auf den Fotoempfänger 23 auf. Abhängig von der Bestrahlungsstärke erzeugt der Fotoempfänger 23 einen Fotostrom I in der Empfangsschaltung 22, die daraus eine beim Ausführungsbeispiel zum Betrag des Fotostroms I proportionale Ausgangsspannung U erzeugt. Die Ausgangsspannung U wird der Steuereinheit 24 als Empfangssignal E bereitgestellt. Die UV-Leuchtdiode 21 ist im Reflexionsprüfmodus inaktiv.

Analog zu dieser Vorgehensweise wird zur Messung eines fluoreszierenden Bereichs 38 die Anlagefläche 17 auf das Objekt 25 aufgelegt, so dass ein fluoreszierender Bereich 38 am Fenster 15 anliegt oder dem Fenster 15 unmittelbar gegenüberliegt (Figur 4).

Nach dem Auswählen des korrekten Fluoreszenzprüfmodus mittels der Bedienschnittstelle 26, abhängig von der Farbe des zu prüfenden fluoreszierenden Bereichs 38, aktiviert die Steuereinheit 24 für die Emissionszeitdauer von beispielsgemäß weniger als 1 Sekunde die UV-Leuchtdiode 21. Die UV-Leuchtdiode 21 strahlt während der Emissionszeitdauer ultraviolettes Licht UVL durch das Fenster 15 auf den zu prüfenden fluoreszierenden Bereich 38 ab. Dort wird das fluoreszierende Material durch das auftreffende ultraviolette Licht UVL angeregt und emittiert fluoreszierendes Licht FL, das durch das Fenster 15 in das Gehäuse 11 eintritt und auf den Fotoempfänger 23 auftrifft. Dadurch wird ein Fotostrom I und ein zum Betrag des Fotostroms I proportionales Empfangssignal E erzeugt und an die Steuereinheit 24 übermittelt.

Die Prüfung bzw. Auswertung in jedem Prüfmodus erfolgt beim Ausführungsbeispiel durch den Vergleich des Empfangssignals E mit wenigstens einem in der Steuereinheit 24 abgespeicherten Referenzwert R. Dieser Referenzwert R entspricht beispielsweise einem Empfangssignal E, das beim Prüfen eines Referenzobjekts gebildet wird. Das Referenzobjekt weist eine bekannte Charakteristik im Hinblick auf die Retroreflexion bzw. die Fluoreszenz auf. Für jeden Prüfmodus ist ein separater Referenzwert R vorgegeben, der bei der Prüfung zum Vergleich herangezogen wird.

Anhand eines vorgegebenen Zusammenhangs, beispielsweise eines linearen Zusammenhangs, kann ein beim Prüfen eines zu prüfenden Objekts 25 erzeugtes Empfangssignal E mit dem hinterlegten Referenzwert R des jeweiligen Prüfmodus verglichen und aus dem Vergleich ein Prüfergebnis erzeugt und ausgegeben werden. Das Prüfergebnis gibt an, ob und/oder in welchem Maß die geprüfte Eigenschaft (Retroreflexion oder Fluoreszenz) des zu prüfenden Objekts der entsprechenden Eigenschaft das durch den Referenzwert des Prüfmodus beschriebenen Referenzobjekts entspricht.

Beim Ausführungsbeispiel wird ein prozentualer Wert P auf der Anzeige 29 ausgegeben, der charakteristisch ist für das Verhältnis des aktuellen Empfangssignals relativ zum Referenzwert des jeweiligen Prüfmodus. Beispielsweise kann der Referenzwert R kann alternativ einen Rückstrahlwert eines retroreflektierenden Materials oder eine Leuchtdichte eines fluoreszierenden Materials eines gerade noch den Vorgaben entsprechenden Referenzobjektes beschreiben, dem eine Prozentangabe zugeordnet wird, beispielsweise 50%. Wenn ein zu prüfendes Objekt 25 ein Empfangssignal E erzeugt, das mindestens dem Referenzwert R entspricht kann anhand der ausgegebenen Prozentangabe P ($\geq$ 50%) erkannt werden, dass das zu prüfende Objekt die vorgegebenen Anforderungen erfüllt. Ist das Empfangssignal E kleiner als der Referenzwert R kann anhand der Prozentangabe P (< 50%) erkannt werden, dass das zu prüfende Objekt die Anforderungen nicht erfüllt.

Somit kann das Handprüfgerät 10 sozusagen einen Schwellwertvergleich ausführen und angeben, ob und/oder in welchem Maß die Anforderungen erfüllt werden oder nicht. Im einfachsten Fall kann lediglich eine das Erfüllen der Vorgaben oder das Nichterfüllen der Vorgaben angebende zweistufige Ausgabe mittels der Bedienschnittstelle 26 erfolgen. Das Ausgeben einer Prozentangabe P hat den Vorteil, dass auch eine Tendenz erkannt werden kann, ob das Objekt 25 die Anforderungen gerade noch erfüllt oder qualitativ noch sehr gut ist. Wie es in Figur 6 veranschaulicht ist, wird beim Ausführungsbeispiel eine lineare Kennlinie angenommen werden, deren Verlauf anhand von bekannten Zusammenhängen oder empirisch ermittelt werden kann. Auch andere Kennlinien können zur Umrechnung des Empfangssignals E in einen angezeigten Wert, beispielsgemäß die Prozentangabe P, verwendet werden.

Beim Kalibrieren des Handprüfgeräts 10 können außerdem zusätzlich zum Referenzwert R ein oder mehrere zusätzliche Kalibrierwerte K erfasst werden. Beispielsweise kann ein Kalibrierwert K an einem Referenzobjekt ermittelt werden, das optimale Eigenschaften im Hinblick auf die Retroreflexion oder die Fluoreszenz aufweist. Durch das Aufnehmen zusätzlicher Kalibrierwerte K kann die Genauigkeit der Kennlinie verbessert werden.

Für jeden Prüfmodus wird zumindest ein Referenzwert R für den Vergleich des Empfangssignals E im jeweiligen Prüfmodus mit dem Referenzwert R hinterlegt. Dies ist deshalb vorteilhaft, weil der Fotoempfänger 23 eine wellenlängenabhängige Charakteristik hat und bei unterschiedlichen Spektren in den verschiedenen Prüfmodi andere Fotoströme I erzeugt. Dadurch lässt sich die Genauigkeit der Auswertung in den verschiedenen Prüfmodi verbessern. Ein schematisch in Figur 6 veranschaulichter Zusammenhang ist daher für jeden Prüfmodus in der Steuereinheit 24 hinterlegt.

Die Erfindung betrifft ein Handprüfgerät 10 und ein Verfahren zum Prüfen eines Objekts 25 mit wenigstens einem retroreflektierenden Bereich 39 und wenigstens einem fluoreszierenden Bereich 38, beispielsweise einem Warnbekleidungsstück 37. Das Handprüfgerät weist eine Weißlicht-Leuchtdiode 20, eine UV-Leuchtdiode 21, einen gemeinsamen Fotoempfänger 23 sowie eine Steuereinheit 24 auf. In einem Reflexionsprüfmodus wird ein reflektierender Bereich 39 mit weißem Licht WL kurzzeitig bestrahlt und auf den Fotoempfänger 23 reflektiert. In wenigstens einem Fluoreszenzprüfmodus wird mittels der UV-Leuchtdiode ultraviolettes Licht UVL auf einen fluoreszierenden Bereich 38 emittiert, der durch die Fluoreszenz fluoreszierendes Licht FL einer entsprechenden Farbe erzeugt, das vom Fotoempfänger 23 empfangen wird. In allen Prüfmodi wird ein Empfangssignal E entsprechend der Bestrahlungsstärke am Fotoempfänger 23 erzeugt und in die Steuereinheit 24 zur Auswertung übermittelt, beispielsweise zur Durchführung eines Schwellenwertvergleichs mit einem vorgegebenem Schwellenwert für das Empfangssignal E. Jeder Prüfmodus kann hierfür vorzugsweise einen separaten vorgegebenen Schwellenwert aufweisen.

Bezugszeichenliste:

| | |
|---|---|
| 10 | Handprüfgerät |
| 11 | Gehäuse |
| 12 | Vorderseite des Gehäuses |
| 13 | Innenraum |
| 14 | Prüfanordnung |
| 15 | Fenster |
| 16 | Platte |
| 17 | Anlagefläche |
| | |
| 20 | Weißlicht-Leuchtdiode |
| 21 | UV-Leuchtdiode |
| 22 | Empfangsschaltung |
| 23 | Fotoempfänger |
| 24 | Steuereinheit |
| 25 | Objekt |
| 26 | Bedienschnittstelle |
| 27 | Rückseite des Gehäuses |
| 28 | Auswahltaste |
| 29 | Anzeige |
| 31 | Betriebsschaltkreis für die Weißlicht-Leuchtdiode |
| 31a | Stromquelle des Betriebsschaltkreises für die Weißlicht-Leuchtdiode |
| 32 | Betriebsschaltkreis für die UV-Leuchtdiode |
| 32a | Stromquelle des Betriebsschaltkreises für die UV-Leuchtdiode |
| 33 | Transmissionsverstärker |
| | |
| 37 | Warnbekleidungsstück |
| 38 | fluoreszierender Bereich |
| 39 | retroreflektierender Bereich |
| A1 | optische Achse des Fotoempfängers |
| A2 | optische Achse der Weißlicht-Leuchtdiode |
| A3 | optische Achse der UV-Leuchtdiode |
| E | Empfangssignal |
| FL | durch Fluoreszenz emittiertes Licht |
| K | Kalibrierwert |
| M | Massepotenzial |
| P | Prozentangabe |
| R | Referenzwert |
| RL | reflektiertes Licht |
| Sr | relative spektrale Sensitivität |
| U | Ausgangsspannung |
| UVL | ultraviolettes Licht |
| WL | weißes Licht |

**Patentansprüche**

1. Handprüfgerät (10), das dazu eingerichtet ist, in einem Reflexionsprüfmodus eine Retroreflexion und in wenigstens einem Fluoreszenzprüfmodus eine Fluoreszenz eines Objekts (25) zu prüfen,

    mit einem Gehäuse (11), an dem eine Bedienschnittstelle (26) zur Auswahl des Reflexionsprüfmodus oder eines Fluoreszenzprüfmodus angeordnet ist,
    wobei das Gehäuse (11) an einer Vorderseite (12) ein zumindest in einem vorgegebenen Wellenlängenbereich lichtdurchlässiges Fenster (15) aufweist,
    wobei in dem Gehäuse (11) eine Prüfanordnung (14) mit einer Weißlicht-Leuchtdiode (20), einer UV-Leuchtdiode (21), einer einen Fotoempfänger (23) aufweisenden Empfangsschaltung (22) und einer Steuereinheit (24) aufweist, wobei der Fotoempfänger (23) nicht sensitiv ist für einfallendes Licht mit einer Wellenlänge von kleiner als 400 nm,
    wobei die Weißlicht-Leuchtdiode (20) und der Fotoempfänger (23) derart angeordnet sind, dass von der Weißlicht-Leuchtdiode (20) emittiertes weißes Licht (WL) durch das Fenster (15) auf das Objekt (25) auftrifft und am Objekt (25) reflektiert wird, und dass das am Objekt (25) reflektierte und durch das Fenster (15) eintretende reflektierte Licht (RL) am Fotoempfänger (23) empfangen wird,

wobei die UV-Leuchtdiode (21) und der Fotoempfänger (23) derart angeordnet sind, dass von der UV-Leuchtdiode (21) emittiertes ultraviolettes Licht (UVL) durch das Fenster (15) auf das Objekt (25) auftrifft und dass vom Objekt (25) aufgrund der Anregung mit ultraviolettem Licht (UVL) durch Fluoreszenz emittiere Licht (FL) am Fotoempfänger (23) empfangen wird,

wobei die Steuereinheit (24) dazu eingerichtet ist, im Reflexionsprüfmodus die Weißlicht-Leuchtdiode (20) und im wenigstens einen Fluoreszenzprüfmodus die UV-Leuchtdiode (21) zur Lichtemission anzusteuern und ein Empfangssignal (E) der Empfangsschaltung (22) auszuwerten, das die Bestrahlungsstärke des auf den Fotoempfänger (23) auftreffenden Lichts beschreibt.

2. Handprüfgerät nach Anspruch 1,
**dadurch gekennzeichnet, dass** das Handprüfgerät (10) dazu eingerichtet ist, einem ersten Fluoreszenzprüfmodus eine Fluoreszenz eines gelb fluoreszierenden Materials eines Objekts (25) zu prüfen und in einem zweiten Fluoreszenzprüfmodus eine Fluoreszenz eines orange fluoreszierenden Materials eines Objekts (25) zu prüfen.

3. Handprüfgerät nach Anspruch 1 oder 2,
**dadurch gekennzeichnet, dass** die Bedienschnittstelle (26) eine Anzeige (29) aufweist, die mit der Steuereinheit (24) kommunikationsverbunden ist.

4. Handprüfgerät nach einem der vorhergehenden Ansprüche, **dadurch gekennzeichnet, dass** die Steuereinheit (24) dazu eingerichtet ist, das Empfangssignal (E) mit einem Referenzwert (R) zu vergleichen.

5. Handprüfgerät nach Anspruch 4,
**dadurch gekennzeichnet, dass** das Vergleichsergebnis angibt, ob das Objekt (25) ausreichend Licht reflektiert oder ausreichend fluoreszierend ist.

6. Handprüfgerät nach Anspruch 4 oder 5,
**dadurch gekennzeichnet, dass** der Referenzwert (R) durch Prüfen wenigstens einer Kalibriereinrichtung oder eines Kalibrierobjekts mit dem Handprüfgerät (10) erzeugt und in der Steuereinheit (24) abgespeichert wird.

7. Handprüfgerät nach Anspruch 6,
**dadurch gekennzeichnet, dass** zusätzlich zum Referenzwert (R) durch Prüfen mit der Kalibriereinrichtung oder mit wenigstens eines weiteren Kalibrierobjekts mit dem Handprüfgerät (10) wenigstens ein weiterer Kalibrierwert (K) erzeugt und in der Steuereinheit (24) abgespeichert wird.

8. Handprüfgerät nach Anspruch 3 und nach einem der Ansprüche 4 bis 7,
**dadurch gekennzeichnet, dass** das Vergleichsergebnis mittels der Anzeige (29) ausgegeben wird.

9. Handprüfgerät nach einem der vorhergehenden Ansprüche, **dadurch gekennzeichnet, dass** das Gehäuse (11) an der Vorderseite (12) eine Anlagefläche (17) aufweist, die dazu eingerichtet ist, während der Prüfung am Objekt (25) angelegt zu werden.

10. Handprüfgerät nach Anspruch 9,
**dadurch gekennzeichnet, dass** das lichtdurchlässige Fenster (15) benachbart zur Anlagefläche (17) angeordnet ist oder zumindest einen Teil der Anlagefläche (17) bildet.

11. Handprüfgerät nach einem der vorhergehenden Ansprüche, **dadurch gekennzeichnet, dass** die Empfangsschaltung (22) einen Transmissionsverstärker (33) aufweist, wobei der Fotoempfänger (23) an einen Eingang des Transmissionsverstärkers (33) angeschlossen ist und das Empfangssignal (E) an einem Ausgang des Transmissionsverstärkers (33) anliegt.

12. Handprüfgerät nach Anspruch 11,
**dadurch gekennzeichnet, dass** der Transmissionsverstärker (33) einen einstellbaren Widerstand (35) aufweist.

13. Handprüfgerät nach einem der vorhergehenden Ansprüche, **dadurch gekennzeichnet, dass** die Weißlicht-Leuchtdiode (20) parallel zu einer optischen Achse (A1) des Fotoempfängers (23) näher am Fenster (15) angeordnet ist als der Fotoempfänger (23).

14. Handprüfgerät nach einem der vorhergehenden Ansprüche, **dadurch gekennzeichnet, dass** die Steuereinheit (24) dazu eingerichtet ist, die Weißlicht-Leuchtdiode (20) und/oder die UV-Leuchtdiode (21) für eine vorgegebene Emissionszeitdauer zu aktivieren.

15. Verfahren zum Prüfen einer Retroreflexion und/oder einer Fluoreszenz eines Objekts (25) mit einem Handprüfgerät (10) aufweisend ein Gehäuse (11), das eine Bedienschnittstelle (26) und an einer Vorderseite (12) ein zumindest in einem vorgegebenen Wellenlängenbereich lichtdurchlässiges Fenster (15) aufweist, wobei in dem Gehäuse (11) eine Prüfanordnung (14) mit einer Weißlicht-Leuchtdiode (20), einer UV-Leuchtdiode (21), einer einen Fotoempfänger (23) aufweisenden Empfangsschaltung (22) und einer Steuereinheit (24) angeordnet ist, wobei der Fotoempfänger (23) nicht sensitiv ist für einfallendes Licht mit einer Wellenlänge von kleiner als 400 nm, mit folgenden Schritten:

- Auswahl des Reflexionsprüfmodus oder eines Fluoreszenzprüfmodus mittels der Bedienschnittstelle (26),
- Ansteuern der Weißlicht-Leuchtdiode (20) im Reflexionsprüfmodus derart, dass die Weißlicht-Leuchtdiode (20) weißes Licht (WL) durch das Fenster (15) auf das Objekt (25) emittiert und der Fotoempfänger (23) das von der Weißlicht-Leuchtdiode (20) emittierte, am Objekt (25) reflektierte und durch das Fenster (15) eintretende reflektierte Licht (RL) empfängt,
- Ansteuern der UV-Leuchtdiode (21) im wenigstens einen Fluoreszenzprüfmodus derart, dass die UV-Leuchtdiode (21) ultraviolettes Licht (UVL) durch das Fenster (15) auf das Objekt (25) emittiert und der Fotoempfänger (23) das vom Objekt (25) durch Fluoreszenz emittiere Licht (FL) empfängt,
- Auswerten eines Empfangssignals (E) der Empfangsschaltung (22), das die Bestrahlungsstärke des auf den Fotoempfänger (22) auftreffenden Lichts beschreibt.

**Claims**

1. Hand-held test device (10) that is configured to test a retro-reflection of an object (25) in a reflection test mode and a fluorescence of the object (25) in at least one fluorescence test mode,

having a housing (11) on which a user interface (26) for selection of the reflection test mode or a fluorescence test mode is arranged, wherein the housing (11) comprises a window (15) at least transparent for light in a predefined wavelength range on a front side (12), wherein a test arrangement (14) having a white light emitting diode (20), a UV-light emitting diode (21), a receiving circuit (22) comprising a photoreceiver (23) and a control unit (24) is provided in the housing (11), wherein the photoreceiver (23) is not sensitive for incident light having a wavelength of less than 400 nm, wherein the white light emitting diode (20) and the photoreceiver (23) are arranged such that white light (WL) emitted by the white light emitting diode (20) through the window (15) impinges on the object (25) and is reflected from the object (25) and that the reflected light (RL) reflected from the object (25) and entering through the window (15) is received on the photoreceiver (23),
wherein the UV-light emitting diode (21) and the photoreceiver (23) are arranged such that ultraviolet light (UVL) emitted from the UV-light emitting diode (21) through the window (15) impinges on the object (25) and that light (FL) emitted, due to fluorescence, from the object (25) because of excitation with ultraviolet light (UVL) is received on the photoreceiver (23),
wherein the control unit (24) is configured to control the white light emitting diode (20) in the reflection test mode and the UV-light emitting diode (21) in the at least one fluorescence test mode for emission of light and to evaluate a receive signal (E) of the receiving circuit (22) that characterizes the irradiance of the light impinging on the photoreceiver (23).

2. Hand-held test device according to claim 1, **characterized in that** the hand-held test device (10) is configured to test in a first fluorescence test mode a fluorescence of a yellow fluorescent material of an object (25) and in a second fluorescence test mode a fluorescence of an orange fluorescent material of an object (25).

3. Hand-held test device according to claim 1 or 2, **characterized in that** the user interface (26) comprises a display (29) that is communicatively coupled with the control unit (24).

4. Hand-held test device according to any of the preceding claims, **characterized in that** the control unit (24) is configured to compare the receive signal (E) with a reference value (R).

5. Hand-held test device according to claim 4, **characterized in that** the comparison result indicates whether the object (25) reflects sufficient light or is sufficiently fluorescent.

6. Hand-held test device according to claim 4 or 5, **characterized in that** the reference value (R) is created by testing of at least one calibration device or a calibration object with the hand-held test device (10) and is stored in the control unit (24).

7. Hand-held test device according to claim 6, **characterized in that** in addition to the reference value (R), at least one additional calibration value (K) is created by testing the calibration device or at least one additional calibration object by means of the hand-held test device (10) and is stored in the control unit (24) .

8. Hand-held test device according to claim 3 and according to any of the claims 4 to 7, **characterized in that** the comparison result is output by means of the display (29) .

**9.** Hand-held test device according to any of the preceding claims, **characterized in that** the housing (11) comprises an abutment surface (17) on the front side (12) that is configured to be brought into abutment with the object (25) during testing.

**10.** Hand-held test device according to claim 9, **characterized in that** the light transparent window (15) is arranged adjacent to the abutment surface (17) or is at least a part of the abutment surface (17).

**11.** Hand-held test device according to any of the preceding claims, **characterized in that** the receiving circuit (22) comprises a transmission amplifier (33), wherein the photoreceiver (23) is connected to an input of the transmission amplifier (33) and the receive signal (E) is provided at an output of the transmission amplifier (33) .

**12.** Hand-held test device according to claim 11, **characterized in that** the transmission amplifier (33) comprises an adjustable resistance 35.

**13.** Hand-held test device according to any of the preceding claims, **characterized in that** parallel to an optical axis (A1) of the photoreceiver (23) the white light emitting diode (20) is arranged closer to the window (15) than the photoreceiver (23).

**14.** Hand-held test device according to any of the preceding claims, **characterized in that** the control unit (24) is configured to activate the white light emitting diode (20) and/or the UV-light emitting diode (21) for a predefined emission period.

**15.** Method for testing a retro-reflection and/or a fluorescence of an object (25) by means of a hand-held test device (10) comprising a housing (11) that comprises a user interface (26) and a window (15) transparent for light at least in a predefined wavelength range on a front side (12), wherein a test arrangement (14) having a white light emitting diode (20), a UV-light emitting diode (21), a receiving circuit (22) comprising a photoreceiver (23) and a control unit (24) is arranged in the housing (11), wherein the photoreceiver (23) is not sensitive for incident light having a wavelength of less than 400 nm, the method comprising the following steps:

- Selection of the reflection test mode or a fluorescence test mode by means of the user interface (26),
- Control of the white light emitting diode (20) in the reflection test mode, such that the white light emitting diode (20) emits white light (WL) through the window (15) onto the object (25) and the photoreceiver (23) receives reflected light (RL) emitted from the white light emitting diode (20), reflected on the object (25) and entering through the window (15),
- Control of the UV-light emitting diode (21) in the at least one fluorescence test mode, such that the UV-light emitting diode (21) emits ultraviolet light (UVL) through the window (15) onto the object (25) and the photoreceiver (23) receives light (FL) emitted by the object (25) due to fluorescence,
- Evaluating a receive signal (E) of the receiving circuit (22) that characterizes the irradiance of the light impinging on the photoreceiver (23).

**Revendications**

**1.** Appareil de test manuel (10) qui est conçu pour vérifier une rétroréflexion, dans un mode de test de réflexion, et une fluorescence d'un objet (25), dans au moins un mode de test de fluorescence,

comprenant un boîtier (11) sur lequel est disposée une interface utilisateur (26) pour sélectionner le mode de test de réflexion ou un mode de test de fluorescence,
le boîtier (11) présentant sur une face avant (12) une fenêtre (15) qui laisse passer la lumière au moins dans une gamme de longueurs d'onde prédéterminée,
sachant que dans le boîtier (11), il est prévu un dispositif de test (14) comportant une diode électroluminescente à lumière blanche (20), une diode électroluminescente UV (21), un circuit de réception (22) présentant un photorécepteur (23), et une unité de commande (24), le photorécepteur (23) n'étant pas sensible à une lumière incidente d'une longueur d'onde inférieure à 400 nm,
sachant que la diode électroluminescente à lumière blanche (20) et le photorécepteur (23) sont disposés de manière à ce que de la lumière blanche (WL) émise par la diode électroluminescente à lumière blanche (20) tombe à travers la fenêtre (15) sur l'objet (25) et soit réfléchie sur l'objet (25), et à ce que la lumière réfléchie sur l'objet (25) et la lumière réfléchie (RL) entrant par la fenêtre (15) soit reçue sur le photorécepteur (23),
sachant que la diode électroluminescente UV (21) et le photorécepteur (23) sont disposés de manière à ce que de la lumière ultraviolette (UVL) émise par la diode électroluminescente UV (21) tombe sur l'objet (25) à travers la fenêtre (15), et à ce que la lumière (FL) émise par l'objet (25) par fluorescence, du fait de l'excitation avec de la lumière ultraviolette (UVL), soit reçue sur le photorécepteur (23),

sachant que l'unité de commande (24) est conçue pour activer la diode électroluminescente à lumière blanche (20), en mode de test de réflexion, et la diode électroluminescente UV (21), dans le mode de test de fluorescence, au nombre d'au moins un, afin qu'elles émettent de la lumière, et pour évaluer un signal de réception (E) du circuit de réception (22), qui décrit l'intensité d'irradiation de la lumière tombant sur le photorécepteur (23).

2. Appareil de test manuel selon la revendication 1, **caractérisé en ce que** l'appareil de test manuel (10) est conçu pour vérifier, dans un premier mode de test de fluorescence, une fluorescence d'un matériau à fluorescence jaune d'un objet (25) et, dans un deuxième mode de test de fluorescence, une fluorescence d'un matériau à fluorescence orange d'un objet (25).

3. Appareil de test manuel selon la revendication 1 ou 2, **caractérisé en ce que** l'interface utilisateur (26) présente un affichage (29) qui est relié du point de vue de la communication à l'unité de commande (24).

4. Appareil de test manuel selon l'une des revendications précédentes, **caractérisé en ce que** l'unité de commande (24) est conçue pour comparer le signal de réception (E) avec une valeur de référence (R).

5. Appareil de test manuel selon la revendication 4, **caractérisé en ce que** le résultat de la comparaison indique si l'objet (25) réfléchit suffisamment de lumière ou s'il est suffisamment fluorescent.

6. Appareil de test manuel selon la revendication 4 ou 5, **caractérisé en ce que** la valeur de référence (R) est générée par vérification d'au moins un dispositif d'étalonnage ou d'un objet d'étalonnage avec l'appareil de test manuel (10) et est enregistrée dans l'unité de commande (24).

7. Appareil de test manuel selon la revendication 6, **caractérisé en ce que**, en plus de la valeur de référence (R), par vérification avec le dispositif d'étalonnage ou avec au moins un objet d'étalonnage supplémentaire, au moins une autre valeur d'étalonnage (K) est générée avec l'appareil de test manuel (10) et enregistrée dans l'unité de commande (24).

8. Appareil de test manuel selon la revendication 3 et selon l'une des revendications 4 à 7, **caractérisé en ce que** le résultat de comparaison est émis à l'aide de l'affichage (29).

9. Appareil de test manuel selon l'une des revendications précédentes, **caractérisé en ce que** le boîtier (11) présente sur la face avant (12) une surface d'appui (17) qui est conçue pour être appliquée contre l'objet (25) pendant le test.

10. Appareil de test manuel selon la revendication 9, **caractérisé en ce que** la fenêtre (15) qui laisse passer la lumière est disposée à proximité de la surface d'appui (17) ou forme au moins une partie de la surface d'appui (17).

11. Appareil de test manuel selon l'une des revendications précédentes, **caractérisé en ce que** le circuit de réception (22) présente un amplificateur de transmission (33), le photorécepteur (23) étant connecté à une entrée de l'amplificateur de transmission (33), et le signal de réception (E) étant appliqué à une sortie de l'amplificateur de transmission (33).

12. Appareil de test manuel selon la revendication 11, **caractérisé en ce que** l'amplificateur de transmission (33) présente une résistance (35) réglable.

13. Appareil de test manuel selon l'une des revendications précédentes, **caractérisé en ce que** la diode électroluminescente à lumière blanche (20) est disposée plus proche de la fenêtre (15) que le photorécepteur (23), parallèlement à un axe optique (A1) du photorécepteur (23).

14. Appareil de test manuel selon l'une des revendications précédentes, **caractérisé en ce que** l'unité de commande (24) est conçue pour activer la diode électroluminescente à lumière blanche (20) et/ou la diode électroluminescente UV (21) pendant une durée d'émission prédéterminée.

15. Procédé de test d'une rétroréflexion et/ou d'une fluorescence d'un objet (25) avec un appareil de test manuel (10) présentant un boîtier (11) qui comporte une interface utilisateur (26) et, sur une face avant (12), une fenêtre (15) qui laisse passer la lumière au moins dans une gamme de longueurs d'onde prédéterminée, sachant que dans le boîtier (11), il est prévu un dispositif de test (14) comportant une diode électroluminescente à lumière blanche (20), une diode électroluminescente UV (21), un circuit de réception (22) présentant un photorécepteur (23), et une unité de commande (24), le photorécepteur (23) n'étant pas sensible à la lumière incidente d'une longueur d'onde inférieure à 400 nm, comprenant les étapes suivantes :

- sélection du mode de test de réflexion ou d'un mode de test de fluorescence à l'aide de l'interface utilisateur (26),
- activation de la diode électroluminescente à lumière blanche (20) en mode de test de réflexion, de manière à ce que la diode électroluminescente à lumière blanche (20) émette de la lumière blanche (W) à travers la fenêtre (15) sur l'objet (25), et le photorécepteur (23) reçoive la lumière (RL) réfléchie émise par la diode électroluminescente à lumière blanche (20), réfléchie sur l'objet (25) et entrant par la fenêtre (15),
- activation de la diode électroluminescente UV (21) dans au moins un mode de test de fluorescence, de manière à ce que la diode électroluminescente UV (21) émette de la lumière ultraviolette (UVL) à travers la fenêtre (15) sur l'objet (25), et le photorécepteur (23) reçoive la lumière (FL) émise par fluorescence par l'objet (25),
- évaluation d'un signal de réception (E) du circuit de réception (22), qui décrit l'intensité d'irradiation de la lumière tombant sur le photorécepteur (23).

Fig.1

Fig.2

Fig.3

Fig.4

Fig.6

Fig.5

Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3351379 A1
- EP 3327627 A

- US 5828460 A
- US 20120229809 A1